## Europäisches Patentamt

## European Patent Office

## Office européen des brevets

(19)

(11) Veröffentlichungsnummer: **0 034 340**
**B1**

(12) **EUROPÄISCHE PATENTSCHRIFT**

(45) Veröffentlichungstag der Patentschrift:
04.01.84

(51) Int. Cl.³: **C 07 D 277/40**

(21) Anmeldenummer: **81100996.8**

(22) Anmeldetag: **12.02.81**

(54) Verfahren zur Herstellung von (2-Aminothiazol-4-yl)-Essigsäurehydrochlorid.

(30) Priorität: **18.02.80 CH 1284/80**

(43) Veröffentlichungstag der Anmeldung:
**26.08.81 Patentblatt 81/34**

(45) Bekanntmachung des Hinweises auf die Patenterteilung:
**04.01.84 Patentblatt 84/1**

(84) Benannte Vertragsstaaten:
**AT BE DE FR GB IT LU NL SE**

(56) Entgegenhaltungen:
**FR - A - 2 199 463
GB - A - 558 956
GB - A - 593 024
GB - A - 1 555 007**

**CHEMISTRY OF HETEROCYCLIC COMPOUNDS, Teil 1,
Band 34, 1979, Wiley, NEW YORK (US)
JOURNAL OF THE AMERICAN CHEMICAL SOCIETY;
Band 63, Heft 11, 7. November 1941, US, W.M. ZIEGLER:
"Preparation of 2-Amino-4-alkylthiazoles from Esters of
Substituted 2-Aminothiazyl-4-acetic Acids", Seiten
2946-2948
BERICHTE DER DEUTSCHEN CHEMISCHEN
GESELLSCHAFT, Band 1, 1896, BERLIN (DE), M.
CONRAD: "Über halogensubstituierte Acetessigester",
Seiten 1042-1048**

(73) Patentinhaber: **LONZA AG, Gampel/Wallis (CH)**

(72) Erfinder: **Huwiler, Alfred, Dr., Sandstrasse 5, Visp
(Kanton Wallis) (CH)**
Erfinder: **Tenud, Leander, Dr., Balfrinstrasse 13, Visp
(Kanton Wallis) (CH)**

(74) Vertreter: **Weinhold, Peter, Dr. et al, Patentanwälte Dr. V.
Schmied-Kowarzik Dipl.-Ing. G. Dannenberg Dr. P.
Weinhold Dr. D. Gudel Dipl.-Ing. S. Schubert
Siegfriedstr. 8, D-8000 München 40 (DE)**

(56) Entgegenhaltungen: (Fortsetzung)
**JOURNAL OF THE AMERICAN CHEMICAL SOCIETY,
Band 66, Heft 5, 9. Mai 1944, US, W.M. ZIEGLER: "Some
4-(Omega-Carboxy-alkyl)-2-aminothiazoles and their
Reaction with Acetylsulfanilyl Chloride", Seiten 744, 745**

Die Akte enthält technische Angaben, die nach dem
Eingang der Anmeldung eingereicht wurden und die
nicht in dieser Patentschrift enthalten sind.

# Verfahren zur Herstellung von (2-Aminothiazol-4-yl)essigsäurehydrochlorid

(2-Aminothiazol-4-yl)essigsäure und deren Derivate stellen Seitenketten semisynthetischer Cephalosporine dar. Aus Justus Liebig's Annalen der Chemie (Bd. 261, 1891) ist ein Verfahren bekannt, bei dem 4-Bromacetessigester mit Thioharnstoff zum (2-Aminothiazol-4-yl)essigsäureesterhydrobromid umgesetzt wird.

Anschliessend wird durch Neutralisation des Hydrochlorids oder Hydrobromids der freie Aminothiazolylessigester hergestellt, der durch Verseifung in die Aminothiazolylessigsäure übergeführt werden kann. Das so erhaltene Produkt ist lichtempfindlich und decarboxyliert in Lösung relativ leicht zum 2-Amino-4-methylthiazol.

Ziel der Erfindung ist es, (2-Aminothiazol-4-yl)essigsäurehydrochlorid auf einfache Weise herzustellen. Erfindungsgemäss wird dies dadurch erreicht, dass man Thioharnstoff in Wasser suspendiert vorlegt und bei Temperaturen von 5 bis 10°C 4-Chloroacetoacetylchlorid — gelöst in einem chlorierten Kohlenwasserstoff — zusetzt, anschliessend die Reaktion bei Temperaturen von 25 bis 30°C zu Ende führt.

Als chlorierter Kohlenwasserstoff wird Tetrachlorkohlenstoff, Chloroform, 1,2-Dichloräthan, vorzugsweise Methylenchlorid, verwendet.

Eine bevorzugte Ausführungsform der Erfindung besteht darin, dass das im chlorierten Kohlenwasserstoff, vorzugsweise Methylchlorid, gelöste 4-Chloracetoacetylchlorid nach Massgabe seines Verbrauchs tropfenweise zum vorgelegten, in Wasser suspendierten Thioharnstoff zugesetzt wird.

Dabei wird die Temperatur zu Beginn der Reaktion bei 5 bis 10°C, vorzugsweise 7 bis 8°C, gehalten und beträgt gegen Ende der Reaktion bis zu 30°C.

Die Menge Wasser, die zur Herstellung der vorzulegenden Thioharnstoffsuspension zur Anwendung kommt, liegt zweckmässig bei 125 bis 250 g Wasser (pro Mol Thioharnstoff).

4-Chloracetoacetylchlorid wird gelöst in chlorierten Kohlenwasserstoffen angewendet. Zweckmässig werden pro Mol 4-Chloracetoacetylchlorid 3 bis 50 mol Lösungsmittel, vorzugsweise 8 bis 25 mol Lösungsmittel, angewendet.

Eine bevorzugte Ausführungsform der Erfindung besteht darin, dass man ein 4-Chloracetoacetylchlorid verwendet, das bei der Chlorierung von Diketen in chloriertem Kohlenwasserstoff, vorzugsweise Methylenchlorid, bei Temperaturen von −10 bis −25°C entsteht. Diese Lösung kann direkt eingesetzt werden.

Das nach dem Verfahren der Erfindung anfallende (2-Aminothiazol-4-yl)essigsäurehydrochlorid ist sowohl in Lösung als auch in fester Form stabil und somit lagerfähig.

*Beispiel*

Zur Herstellung der Ausgangskomponente des vorliegenden Verfahrens, des 4-Chloracetoacetylchlorids, werden in einem Doppelmantelkolben 187,7 g Methylenchlorid, zusammen mit 18,6 g Diketen, vorgelegt und auf −25°C abgekühlt. In diese Lösung wird sodann bei einer Temperatur von −20 bis −25°C Chlor eingeleitet. Parallel wird in einem Rundkolben eine Suspension von 15,2 g Thioharnstoff in 30,0 g Wasser vorbereitet und diese auf +5°C abgekühlt.

In die vorgelegte, auf +5 bis +7°C abgekühlte Suspension von Thioharnstoff wird aus dem Tropftrichter der Apparatur die vorbereitete 4-Chloracetoacetylchloridlösung unter stetem Rühren bei +7 bis +8°C innert 25 min zugetropft und nach Beendigung der Zugabe noch 30 min bei +5 bis +7°C weitergerührt, dann das Kühlbad entfernt und das Rühren weitere 60 min fortgesetzt, wobei ein Temperaturanstieg auf +26 bis +27°C eintritt. Anschliessend wird das Reaktionsgemisch zur Ausfällung des (2-Aminothiazol-4-yl)essigsäurehydrochlorids in den Kühlschrank gestellt.

Man isoliert als Ausbeute 33,6 g (2-Aminothiazol-4-yl)essigsäurehydrochlorid als farblose Kristalle mit einem Schmelzpunkt von 151,4 bis 151,9°C, entsprechend einer Ausbeute von 78,5%, bezogen auf das eingesetzte Diketen.

## Patentansprüche

1. Verfahren zur Herstellung von (2-Aminothiazol-4-yl)essigsäurehydrochlorid, dadurch gekennzeichnet, dass man Thioharnstoff in Wasser suspendiert vorlegt und bei Temperaturen von 5 bis 10°C 4-Chloracetoacetylchlorid — gelöst in einem chlorierten Kohlenwasserstoff — zusetzt, anschliessend die Reaktion bei Temperaturen von 25 bis 30°C zu Ende führt.

2. Verfahren nach Patentanspruch 1, dadurch gekennzeichnet, dass man als chlorierten Kohlenwasserstoff Methylenchlorid verwendet.

3. Verfahren nach den Patentansprüchen 1 und 2, dadurch gekennzeichnet, dass man das im chlorierten Kohlenwasserstoff gelöste 4-Chloracetoacetylchlorid nach Massgabe seines Verbrauchs zusetzt.

4. Verfahren nach den Patentansprüchen 1 bis 3, dadurch gekennzeichnet, dass man eine 4-Chloracetoacetylchloridlösung verwendet, die bei der Chlorierung von Diketen im chlorierten Kohlenwasserstoff bei Temperaturen von −10 bis −25°C anfällt.

## Claims

1. A process for the production of (2-aminothiazole-4-yl) acetic acid hydrochloride, characterized by introducing at first thio-urea in aqueous suspension, adding thereto at temperatures of from 5 to 10°C 4-chloroacetoacetylchloride dissolved in a chlorinated hydrocarbon, and subsequently completing the reaction at temperatures of from 25 to 30°C.

2. A process according to claim 1, characterized in using as a chlorinated hydrocarbon methylene chloride.

3. A process according to claims 1 and 2, characterized in adding the 4-chloroacetoacetylchloride dissolved in chlorinated hydrocarbon according to the consumed amount.

4. A process according to claims 1 to 3, characterized by using a 4-chloroacetoacetylchloride solution which results from the chlorination of diketene in the chlorinated hydrocarbon at temperatures of from −10 to −25°C.

**Revendications**

1. Procédé pour la préparation du chlorhydrate de l'acide (2-aminothiazol-4-yl)acétique, caractérisé en ce que l'on met au préalable de la thio-urée en suspension dans l'eau et en ce qu'on ajoute, à des températures de 5 à 10°C, du chlorure de 4-chloroacétoacétyle dissous dans un hydrocarbure chloré, puis en ce qu'on conduit la réaction jusqu'à son terme à des températures de 25 à 30°C.

2. Procédé selon la revendication 1, caractérisé en ce que l'on utilise comme hydrocarbure chloré du chlorure de méthylène.

3. Procédé selon les revendications 1 et 2, caractérisé en ce que l'on ajoute le chlorure de 4-chloroacétoacétyle dissous dans l'hydrocarbure chloré au fur et à mesure de sa consommation.

4. Procédé selon les revendications 1 à 3, caractérisé en ce que l'on utilise une solution de chlorure de 4-chloroacétoacétyle qui est obtenue lors de la chloration du dicétène dans l'hydrocarbure chloré à des températures de −10 à −25°C.